(19) Europäisches Patentamt
European Patent Office
Office européen des brevets

(11) **EP 3 328 820 B1**

(12) **EUROPÄISCHE PATENTSCHRIFT**

(45) Veröffentlichungstag und Bekanntmachung des Hinweises auf die Patenterteilung:
**30.09.2020 Patentblatt 2020/40**

(21) Anmeldenummer: **16744365.4**

(22) Anmeldetag: **26.07.2016**

(51) Int Cl.:
***C07C 29/149*** *(2006.01)* ***C07C 31/20*** *(2006.01)*

(86) Internationale Anmeldenummer:
**PCT/EP2016/067706**

(87) Internationale Veröffentlichungsnummer:
**WO 2017/017074 (02.02.2017 Gazette 2017/05)**

# (54) VERFAHREN ZUR HERSTELLUNG VON MONOETHYLENGLYKOL

METHOD FOR THE PREPARATION OF MONOETHYLENE GLYCOL

PROCEDE DE FABRICATION DE MONO-ETHYLENE GLYCOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2015 EP 15178919**

(43) Veröffentlichungstag der Anmeldung:
**06.06.2018 Patentblatt 2018/23**

(73) Patentinhaber: **BASF SE**
**67056 Ludwigshafen am Rhein (DE)**

(72) Erfinder:
- **DUEFERT, Alexander**
  **67063 Ludwigshafen (DE)**
- **PINKOS, Rolf**
  **67098 Bad Duerkheim (DE)**
- **REISER, Michael**
  **67655 Kaiserslautern (DE)**
- **BRUEGGEMANN, Philipp**
  **91052 Erlangen (DE)**
- **THEIS, Gerhard**
  **67133 Maxdorf (DE)**

(74) Vertreter: **BASF IP Association**
**BASF SE**
**G-FLP-C006**
**67056 Ludwigshafen (DE)**

(56) Entgegenhaltungen:
**CN-A- 101 475 443** **US-A1- 2010 179 356**

EP 3 328 820 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein Verfahren zur Herstellung von Monoethylenglykol. Monoethylenglykol, Formel HO-$CH_2$-$CH_2$-OH, wird im Folgenden als MEG abgekürzt oder kurz Ethylenglykol genannt.

Ethylenglykol ist eine wichtige Plattformchemikalie, welche für die Herstellung von Polyestern, als Frostschutzmittel, Schmiermittel, Weichmacher, und vieles Weiteres eingesetzt wird. Im Jahr 2010 lag die globale Produktion bei ca. 20 Millionen Tonnen, mit einem projizierten jährlichen Wachstum von 5-10 % pro Jahr. Die Hauptproduktionsroute basierte auf der wässrigen Hydrolyse von Ethylenoxid zu MEG, welches aus petrochemischen Quellen gewonnen wird. Ein weiterer wichtiger Prozess ist die Hydrierung von Oxalaten.

Die Herstellung von MEG durch Hydrierung von Derivaten der Oxalsäure (Formel: HOOC-COOH) ist mehrfach beschrieben. Abgesehen von Forschungsarbeiten basieren auch die aktuell durchgeführten industriellen Verfahren auf der Hydrierung von Oxalsäurestern, meistens Dimethyloxalat (DMO) an Kupfer-Katalysatoren in der Gasphase. Schwerpunkt ist die Hydrierung von durch Umsetzung von Methanol und CO hergestelltem Dimethyloxalat nach einem ursprünglich von Ube entwickeltem Verfahren (US 4,453,026 A, Ube Industries Ltd.). In keinem Dokument ist erwähnt, wie man ausgehend von Oxalaten MEG in hoher Ausbeute und geringen Mengen an störenden Nebenkomponenten bei hohem Druck herstellt.

C3- oder C4-Verbindungen, die beispielsweise 1,2-Propandiol, 1,2-Butandiol, Butanal, Propanal, aber auch C2-Körper wie Acetaldehyd, enthalten können, sind im Austrag unerwünscht, da sie nur schwer vom Produkt abzutrennen sind, in das Reinprodukt gelangen und sich negativ auf die Farbstabilität des Ethylenglykols oder dessen Folgeprodukte auswirken können. Dimethyloxalat kann sich außerdem in C1-Körper wie CO oder $CO_2$ zersetzen, wodurch die Selektivität und Rentabilität des Gesamtprozesses gesenkt wird.

L. Zhuxia et al., Huaxue fanying gongcheng yu gongyi (Chemical Reaction Engineering and Technology), 2004, 20, Seiten 121-128, beschreibt die Gasphasenreaktion von DMO mit Wasserstoff an auf $SiO_2$-geträgerten Kupferkatalysatoren bei 523 bis 623 K (250 bis 350 °C) und 1,5 bis 5,0 MPa (loc. cit. Fig. 5), entsprechend 15 bis 50 bar. Das molare $H_2$/DMO-Verhältnis lag im Bereich von 25 bis 160 (loc. cit. Fig. 4). Es wurde gefunden, dass ein hoher Druck und eine hohes molares $H_2$/DMO-Verhältnis positiv für die Selektivität zu MEG ist, ein niedriges molares $H_2$/DMO-Verhältnis jedoch zu vermehrter Methylglykolat-Bildung führt. Ein gleichzeitiges Fahren bei niedrigem molaren $H_2$/DMO-Verhältnis bei hohem Druck wurde nicht untersucht, auch wurde die Bildung von störenden Nebenkomponenten nicht erwähnt.

Y. Wang et al., Catal. Sci. Technol., 2012, 2, Seiten 1637-1639, erwähnt für eine Gasphasenhydrierung (loc. cit. Seite 1637, rechte Spalte, 3. Absatz) von DMO zur MEG bei 453 K (180 °C) die Bildung von Ethanol, 1,2-Propandiol und 1,2-Butandiol als unerwünschte Nebenkomponenten. Es wird keine Aussage zur Zersetzung von DMO zu CO oder $CO_2$ gemacht, auch findet die Hydrierung bei niedrigem Druck von 3,0 MPa (30 bar) und einem hohen $H_2$-Überschuss ($H_2$/DMO = 80:1) statt (loc. cit. Table 1).

Die metallkatalysierte Flüssigphasenhydrierung von Dialkylestern der Oxalsäure zu Ethylenglykol bei Temperaturen im Bereich von 200 bis 275 °C wird in US 2,060,880 (E. I. du Pont de Nemours & Comp.) beschrieben. Für Oxalsäurediethylester wurde ein Druck im Bereich von 200 bis 1000 atm (202,65 bis 1013,25 bar) und eine Temperatur im Bereich von 240 bis 260 °C untersucht (Katalysator: Kupferchromit). Erst ab einem hohen Druck von mind. 600 atm (405,3 bar) wird ein praktisch vollständiger Umsatz des Diesters erzielt. Die Ausbeute an MEG lag bei 600 atm bei 80-85 % und bei 1000 atm bei 85 %. Als mögliche Nebenprodukte werden lediglich Ethanol und Ether benannt, auch wird das Verfahren diskontinuierlich durchgeführt.

In der Offenlegungsschrift DE 38 43 956 A1 (Hüls AG) wird von einer kontinuierlichen Hochdruck-Flüssigphasenhydrierung von Dicarbonsäurediestern zu alpha-omega-Diolen an KupferChromit-Katalysatoren berichtet. Die Umsetzungen erfolgen bei 120 bis 220 °C und 50 bis 400 bar, wobei die Ausbeute z.B. im Fall von Hexan-1,6-diol bei 300 bar 93 % betrug. Die Herstellung von MEG aus Oxalsäurediestern und damit verbundene Nebenkomponenten werden jedoch nicht erwähnt.

In CN 101 475 443 A (China Petrochem. Corp.) wird von der Herstellung von MEG aus Oxalaten an Kupfer-Kontakten im Mitteldruckbereich von 0,1 bis 10,0 MPa (1 bis 100 bar) und im Temperaturbereich von 120 bis 300 °C berichtet. Die Fahrweise wurde gewählt, um eine lange Katalysatorstandzeit und kurze Regenerationszeit zu bewirken. Die Verwendung höherer Drücke und ihre Auswirkung auf die Selektivität wird nicht erwähnt. Das molare $H_2$/Ester-Verhältnis wird im Bereich von 5 bis 300 angegeben. In den Beispielen liegt die Temperatur im Bereich von 180 bis 270 °C, der Druck im Bereich von 0,8 bis 4,0 MPa (8 bis 40 bar) und das molare $H_2$/Ester-Verhältnis im Bereich von 60 bis 150. Trotz des hohen Wasserstoff-Überschusses konnte in den angegebenen Beispielen bei vollständigem Umsatz nur eine Selektivität im Bereich von 81 bis 96 % realisiert werden.

In H. Yue et al., Chem. Soc. Rev., 2012, 41, Seiten 4218-4244, wird die homogen- und heterogen-metallkatalysierte Hydrierung von DMO referiert.

Für die diskontinuierliche Hydrierung in flüssiger Phase wird zitiert, dass für eine effiziente Umsetzung unter Homogenkatalyse ein hoher Druck von z.B. 200 bar notwendig ist (Seite 4223, rechte Spalte). Es wird zwar auf die

Bildung von Nebenkomponenten durch Veresterungs-, Umesterungs- und Decarboxylierungsreaktionen hingewiesen, mögliche Nebenkomponenten werden aber nicht näher beschrieben. Spezielle Ru-Homogenkatalysatoren ermöglichen die MEG-Herstellung aus DMO auch unter milden Bedingungen von 80-100 bar $H_2$ und 120 °C. Weiterhin werden Ergebnisse zur kontinuierlichen DMO-Hydrierung in der Gasphase an Kupfer-Heterogenkatalysatoren zitiert (Seiten 4224-4225). Bei einem molaren $H_2$/DMO-Verhältnis im Bereich von 50 bis 200, 463 bis 516 K (190 bis 243 °C) und 20 bis 30 bar werden MEG-Ausbeuten bis zu 99 % erreicht.

H.T. Teunissen et al., Chem. Commun., 1997, Seiten 667-668, beschreibt die diskontinuierliche homogene Hydrierung von DMO zu MEG unter milden Bedingungen (70 bar $H_2$, 100 °C) an Rutheniumkatalysatoren. Die Ausbeuten betragen nur bei ausgewählten Ru-Katalysatoren mit bestimmten Liganden bis zu 95 %.

US 2010/0179356 A1 (J. Liu et al.) betrifft die Herstellung von Ethylenglykol durch Hydrierung von Oxalat(en) in der Gasphase bei einem Druck im Bereich von 2 bis 100 bar umfassend a) eine erste Reaktionszone mit einem ersten kupferhaltigen Katalysator und b) eine zweite Reaktionszone mit einem zweiten kupferhaltigen Katalysator.

Der vorliegenden Erfindung lag die Aufgabe zugrunde, unter Überwindung von Nachteilen des Stands der Technik, ein verbessertes wirtschaftliches Verfahren zur Herstellung von MEG bereitzustellen, in dem das MEG in hoher Ausbeute, insb. Raum-Zeit-Ausbeute, und Reinheit erhalten wird. Das Verfahren sollte bei hohem Umsatz durchgeführt werden können, insbesondere auch in kontinuierlicher Fahrweise, und eine hohe Selektivität aufweisen, d.h. z.B. eine möglichst geringe Zersetzung des Edukts und eine möglichst geringfügige Bildung von Nebenkomponenten, darunter auch potentiell farbgebenden Nebenkomponenten. Der eingesetzte Katalysator sollte unter den Verfahrensbedingungen eine hohe Lebensdauer (Standzeit) aufweisen. Das Herstellverfahren sollte zudem besonders einfach und wirtschaftlich sein.

Demgemäß wurde ein Verfahren zur Herstellung von Monoethylenglykol (MEG) durch metallkatalysierte Umsetzung eines Oxalsäuredialkylesters der Formel I

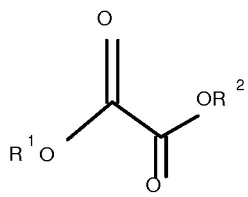

(I),

in der $R^1$ und $R^2$, jeweils unabhängig voneinander, Methyl, Ethyl, n-Propyl oder iso-Propyl, bedeuten,
mit Wasserstoff ($H_2$) gefunden, welches dadurch gekennzeichnet ist, dass man den Oxalsäuredialkylester (I) als Schmelze oder gelöst in einem Lösungsmittel einsetzt, Oxalsäuredialkylester (I) und $H_2$ in einem molaren Verhältnis $H_2$ : Oxalsäuredialkylester (I) im Bereich von 4,0 bis 30 einsetzt, und die Umsetzung kontinuierlich in einem Reaktor bei einer Querschnittsbelastung von $\geq$ 10 m/s, einer Temperatur im Bereich von 150 bis 270 °C, einem Druck im Bereich von 150 bis 390 bar und in Gegenwart eines chromfreien Heterogenkatalysators, der Kupfer enthält, durchführt.

Die chemische Umsetzung entspricht der folgenden Reaktionsgleichung:

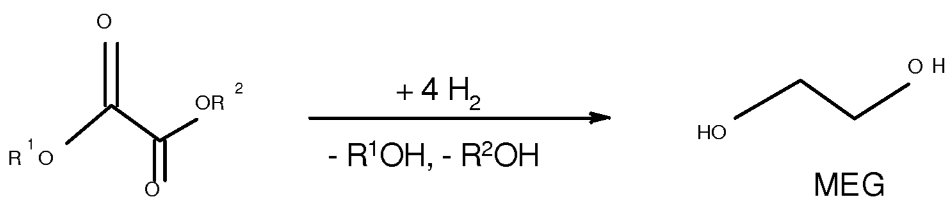


Bei den Resten $R^1$ und $R^2$ handelt es sich, jeweils unabhängig voneinander, um Methyl, Ethyl, n-Propyl oder iso-Propyl.

Beispielsweise ist $R^1 = R^2$ = Ethyl. Besonders bevorzugt ist $R^1 = R^2$ = Methyl, das zu hydrierende Edukt also Dimethyloxalat (DMO).

Erfindungsgemäß wurde erkannt, dass sich durch das anspruchsgemäße Verfahren ein hoher Umsatz an Ester (I) von besonders $\geq$ 90 % bei geringer Zersetzung des Esters (I) von besonders < 10 %, und hoher Selektivität zum Ethylenglykol realisieren lässt.

Die Selektivität (definiert wie unten bei den Beispielen beschrieben) für Ethylenglykol, bezogen auf eingesetztes Dialkyloxalat I, beträgt über die gesamte Laufzeit des Hydrierkatalysators besonders über 90 %, insbesondere über 95 %, ganz besonders $\geq$ 96 %.

Zudem erweist sich das Flüssigphasen-Verfahren als vorteilhaft gegenüber den GasphasenVerfahren des Stands der Technik, da insbesondere energetische und verfahrenstechnische Vorteile erzielt werden (kein Verdampfungsschritt für das Edukt, kein Kondensationsschritt für den Reaktoraustrag).

Der Oxalsäureester I kann gelöst in einem Lösungsmittel eingesetzt werden. Dazu kann der Oxalsäureester I als Feststoff in das gewünschte Lösungsmittel eingebracht werden. Dies ist von Vorteil, wenn in der vorherigen Herstellung von Oxalsäureester I Nebenprodukte anfallen, die hemmend oder schädigend auf den Hydrierprozess wirken können oder durch die Hydrierung in Verbindungen überführt werden, die sich aufgrund ihrer physikalischen Eigenschaften nicht oder nur schwer von Ethylenglykol trennen lassen und sich durch zwischenzeitliches Isolieren von Oxalsäureester I, z.B. DMO, als Feststoff abreichern lassen.

Alternativ kann der Oxalsäureester I auch als gasförmiger Strom in ein gewünschtes Lösungsmittel eingebracht werden. Dies ist z.B. dann von Vorteil, wenn eine Koppelproduktion von Oxalsäureester I, z.B. DMO, und MEG in zwei hintereinandergeschalteten Anlagen zur Herstellung von Oxalsäureester I, z.B. DMO, und einer direkten Weiterverarbeitung zu MEG angestrebt wird und ein variabler Teil an Oxalsäureester I, z.B. DMO, ausgeschleust werden soll.

Der Oxalsäureester I kann im erfindungsgemäßen Verfahren auch als reine oder mit geringen Mengen, z.B. 0,1 bis 10 Gew.-%, an Lösungsmittel(n) versetzte Schmelze eingesetzt werden. Auf diese Weise kann die Menge an Lösungsmittel(n) im Verfahren minimiert werden, was sich für eine abschließende Abtrennung des MEGs als positiv erweisen kann, da die Kreisströme an Lösungsmittel(n) so deutlich kleiner werden.

Beispiele für verwendbare Lösungsmittel, die unter den Reaktionsbedingungen inert sind, sind aliphatische oder aromatische Alkohole, Kohlenwasserstoffe, Ether auf Basis Ethylenoxid und/oder Propylenoxid, aliphatische oder aromatische Ester oder Carbonate, Wasser, substituierte Phenolderivate. Es können auch Mischungen von zwei oder mehr Lösungsmitteln verwendet werden, sofern dies gewünscht ist. Bevorzugte Lösungsmittel sind Methanol, Ethanol, n-Propanol, iso-Propanol und/oder Ethylenglykol. Methanol und Ethylenglykol sind besonders bevorzugt.

In einer besonderen Ausführungsform wird der Oxalsäureester I als Schmelze zugefahren, wobei eine Vermischung des Edukts I mit einem Rohprodukt-Teilstrom (Flüssig-Umlauf am Reaktor) zu einer Lösung des Edukts I, z.B. DMO in MEG/MeOH, als Feed des Reaktors führt.

Die Umsetzung wird bei einer Temperatur im Bereich von 150 bis 270 °C, bevorzugt bei 170 bis 260 °C, weiter besonders bei einer Temperatur im Bereich von 180 bis 250 °C, ganz besonders bei einer Temperatur im Bereich von 190 bis 240 °C, durchgeführt.

Die Umsetzung wird bei einem Druck im Bereich von 150 bis 390 bar, bevorzugt bei einem Druck im Bereich von 160 bis 290 bar, weiter besonders bei einem Druck im Bereich von 180 bis 270 bar, durchgeführt.

Bevorzugt werden im erfindungsgemäßen Verfahren Oxalsäuredialkylester (I) und $H_2$ in einem molaren Verhältnis $H_2$ : Oxalsäuredialkylester (I) im Bereich von 4,2 bis 20, besonders im Bereich von 4,3 bis 15, eingesetzt.

Bei dem molaren Verhältnis $H_2$ : Oxalsäuredialkylester (I) handelt es sich um das molare Verhältnis des Frisch-Wasserstoffs zu Frisch-Oxalsäuredialkylester (I), also der beiden für die Umsetzung kontinuierlich zugeführten Edukte.

Sollte ein Nachreaktor eingesetzt werden, kann auch mit substöchiometrischen Mengen an Wasserstoff im Hauptreaktor gefahren werden.

Die Umsetzung wird kontinuierlich in einem Reaktor bei einer Querschnittsbelastung (Q) von ≥ 10 m/s durchgeführt.

Die Querschnittsbelastung (= Volumenstromdichte) Q ist wie folgt definiert:

$$Q = \frac{\dot{V}}{A} = \frac{Volumendurchsatz}{Querschnittsfläche\ des\ Reaktors}$$

Die Einheit von Q lautet m/s.

Die Umsetzung wird kontinuierlich in einem Reaktor bei einer bevorzugten Querschnittsbelastung (Q) im Bereich von ≥ 10 bis 1000 m/s, weiter bevorzugt im Bereich von > 10 bis 500 m/s, weiter besonders im Bereich von 15 bis 400 m/s, ganz besonders im Bereich von 20 bis 300 m/s, durchführt.

Die erfindungsgemäße kontinuierliche Umsetzung erfolgt bevorzugt bei einer Katalysatorbelastung im Bereich von 0,01 bis 5,0 kg Oxalsäuredialkylester (I) • $Liter_{Kat.}^{-1}$ • $h^{-1}$, besonders bevorzugt bei einer Katalysatorbelastung im Bereich von 0,1 bis 2,0 kg Oxalsäuredialkylester (I) • $Liter_{Kat.}^{-1}$ • $h^{-1}$, weiter besonders bevorzugt bei einer Katalysatorbelastung im Bereich von 0,2 bis 1,5 kg Oxalsäuredialkylester (I) • $Liter_{Kat.}^{-1}$ • $h^{-1}$.

In einer bevorzugten Ausführungsform des erfindungsgemäßen Verfahrens wird die Katalysatorbelastung derart einstellt, dass der Umsatz an Oxalsäuredialkylester (I) ≥ 90 %, bevorzugt ≥ 95 %, besonders bevorzugt ≥ 96 %, beträgt.

Als Reaktoren für die erfindungsgemäße Umsetzung (Hydrierung des Oxalsäureesters I) kommen dem Fachmann bekannte Typen zum Einsatz. Beispiele hierfür sind Schachtreaktoren, besonders Rohrreaktoren, und Rohrbündelreaktoren, usw. Es kann in einem Reaktor hydriert werden oder in mehreren, parallel oder hintereinander angeordnet, auch mehrere Typen miteinander kombiniert.

Bevorzugt wird die Hydrierung mit Teilumsatz in einem ersten Reaktor mit Flüssigumlauf und Nachreaktor betrieben. Hierfür wird das Rohprodukt bei einem oder mehreren hintereinander oder parallel angeordneten Reaktoren zwischen den Reaktoren oder nach den Reaktoren über eine Pumpe in einen weiter vorne im Prozess liegenden Reaktor rückgeführt, wobei der Umlaufstrom bevorzugt dazu verwendet wird, die Reaktionswärme im Umlauf über einen Wärmetauscher abzuführen. Der durch die Hydrierung und Verlusten über Abgas verbrauchte Wasserstoff wird entsprechend ergänzt. Das Gewichtsverhältnis von Umlauf zu Austrag beträgt besonders 1 - 100 zu 1, bevorzugt 3 - 50 zu 1, besonders bevorzugt 5 - 20 zu 1, z.B. 10 zu 1.

Bevorzugt ist, dass man die Umsetzung in einem Reaktor kontinuierlich durchführt und der Reaktor mit einem Umlauf betrieben wird, indem ein Teil des Reaktoraustrags vom Reaktorausgang zum Reaktoreingang zurückgeführt wird.

Sollte eine Kombination aus Haupt- und Nachreaktor verwendet werden, wird bevorzugt ein Kreisgasstrom nach dem Hauptreaktor zurückgeführt und die Nachhydrierung mit Frischwasserstoff gefahren. Alternativ kann ohne Kreisgasstrom gefahren werden, wobei zwischen einzelnen Reaktoren Frischgas neu eingespeist werden kann. Eine zusätzliche Zwischendosierung an Wasserstoff im Reaktor ist ebenfalls möglich.

Bei länger laufenden Prozessen zur Herstellung technischer Produktmengen an Ethylenglykol erfolgt bevorzugt eine Ausschleusung zur Vermeidung von Aufpegelungen von unerwünschten Nebenkomponenten. Deshalb ist es bevorzugt, diesen Ausschleusestrom, z.B. in einer weiteren Destillationseinrichtung, weiter aufzuarbeiten.

Mögliche Zersetzungsprodukte von Oxalsäureester I, CO und $CO_2$, können sich ebenfalls aufpegeln, weswegen ein Teil des Kreisgasstroms in diesem Fall bevorzugt ausgeschleust wird, um ein Anreichern an Zersetzungsprodukten zu vermeiden.

Das erfindungsgemäße Verfahren wird in Gegenwart eines chromfreien Heterogenkatalysators, der Kupfer enthält, durchführt.

Unter einem chromfreien Heterogenkatalysator ist zu verstehen, dass bei der Herstellung des Katalysators kein Chrom (Cr) jedweder Oxidationsstufe zugesetzt wird. Herstellbedingt oder aufgrund von Verunreinigungen der Katalysatoredukte soll der Katalysator weniger als 0,5 Gew.-%, bevorzugt weniger als 0,1 Gew.-%, besonders bevorzugt weniger als 0,01 Gew.-% Chrom jedweder Oxidationsstufe enthalten.

Die zur Hydrierung genutzten Katalysatoren enthalten als Aktivkomponente zumindest Cu, daneben können als weitere Hydriermetalle eines oder mehrere Elemente aus der Gruppe Ru, Re, Co, La, Mo, W, Ti und Zr enthalten sein. Bevorzugt ist z.B. ein Gemisch aus Cu und einem Oxid eines weiteren Elements aus der Gruppe La, Mo, W, Ti und Zr. Bei solch einer Mischung von Kupfer und einem Metalloxid ist die Verwendung von Lanthan besonders bevorzugt.

Der Gew.-%-Anteil des Hydriermetalls bzw. der Hydriermetalle (berechnet als Element/e), z.B. des Kupfers, am Gesamtgewicht des Katalysators liegt bevorzugt im Bereich von 0,5 bis 85, weiter bevorzugt im Bereich von 10 bis 80, besonders weiter bevorzugt im Bereich von 25 bis 65.

Der Gew.-%-Anteil des Oxids aus der Gruppe La, Mo, W, Ti und Zr, z.B. des Lanthanoxids ($La_2O_3$), am Gesamtgewicht des Katalysators liegt bevorzugt im Bereich von 0,01 bis 30, weiter bevorzugt im Bereich von 0,5 bis 20, weiter besonders im Bereich von 3 bis 15.

Das bzw. die Hydriermetall/e ist/sind bevorzugt auf einem Trägersystem aufgebracht. Geeignete Träger enthalten Oxide oder bestehen aus Oxiden basierend auf B, Al, Si, Ti, Zr, La, Ce oder Cr, z.B. Aluminiumoxid oder Zirkoniumdioxid, oder Kohlenstoff, beispielsweise in Form von Aktivkohle. Ein weiterer Träger, der nicht oxidisch vorliegt, ist beispielsweise SiC.

Der Gew.-%-Anteil des Trägermaterials, z.B. des Aluminiumoxids, am Gesamtgewicht des Katalysators liegt besonders im Bereich von 15 bis 98, weiter besonders im Bereich von 19,5 bis 89,5, ganz besonders im Bereich von 32 bis 72.

In einem bevorzugten Beispiel enthält der Katalysator, bezogen auf das Gesamtgewicht des Katalysators, im Bereich von 10 bis 80 Gew.-%, besonders im Bereich von 25 bis 65 Gew.-%, Kupfer, im Bereich von 0,5 bis 20 Gew.-%, besonders im Bereich von 3 bis 15 Gew.-%, Lanthanoxid und im Bereich von 19,5 bis 89,5 Gew.-%, besonders im Bereich von 32 bis 72 Gew.-%, Aluminiumoxid.

Die Herstellung der Katalysatoren wird beispielsweise durch Tränken von Aktivmetallvorläufern, wie entsprechende Metallsalz-Lösungen, z.B. eine Cu-Salzlösung, auf dem entsprechenden Träger erreicht. Geeignet sind auch Fällkatalysatoren, bei denen die Aktivkomponenten auf einen Träger aufgefällt werden oder zusammen mit dem Trägermaterial aus gelösten Vorstufen desselben gefällt werden. Nach Trocknung und ggf. Calzination des Katalysatormaterials wird bevorzugt vor Beginn der Hydrierung der Katalysator mit Wasserstoff aktiviert.

Die Katalysatoren sind im Allgemeinen Formkörper mit einer durchschnittlichen Größe, die über einem Millimeter liegt. Bevorzugt werden Stränge, Tabletten, Sternstränge, Triloben, Hohlkörper usw. verwendet.

Die Katalysatorschüttdichte liegt besonders im Bereich von 0,5 bis 2,0 g/ml.

In einer besonderen Ausführungsform des erfindungsgemäßen Verfahrens wird die Hydrierung des Oxalsäureesters I mittels eines Katalysator-Formkörpers, dessen Vorläufer gemäß einem Verfahren herstellbar ist, in dem

(i) ein oxidisches Material, umfassend Kupferoxid, Aluminiumoxid und Lanthanoxid bereitgestellt wird,
(ii) dem oxidischen Material pulverförmiges metallisches Kupfer und/oder Kupferblättchen und optional Graphit zugegeben wird,
(iii) das aus ii resultierende Gemisch zu einem Formkörper verformt wird,
durchgeführt,

wobei das oxidische Material erhältlich ist durch simultanes oder nacheinander erfolgendes Fällen der Komponente Kupferoxid, der Komponente Aluminiumoxid und der Komponente Lanthanoxid und anschließendes Trocknen und Calcinieren
und nach der Verformung nach Schritt iii der Katalysator-Formkörper nochmals calciniert wird.

Weiter besonders sind diese Katalysator-Förmkörper, die im Folgenden auch noch weiter ausgeführt werden, dadurch gekennzeichnet, dass das oxidische Material

(a) Kupferoxid mit einem Anteil im Bereich von $50 \leq x \leq 80$ Gew.-%, vorzugsweise $55 \leq x \leq 75$ Gew.-%, jeweils berechnet als CuO,
(b) Aluminiumoxid mit einem Anteil im Bereich von $15 \leq y \leq 35$ Gew.-%, vorzugsweise $20 \leq y \leq 30$ Gew.-%, und
(c) Lanthanoxid mit einem Anteil im Bereich von $2 \leq z \leq 20$ Gew.-%, bevorzugt $3 \leq z \leq 15$ Gew.-%, weiter bevorzugt $3{,}5 \leq z \leq 10$ Gew.-%,

jeweils bezogen auf das Gesamtgewicht des oxidischen Materials nach Calcinierung, wobei gilt: $80 \leq x + y + z \leq 100$, insbesondere $95 \leq x + y + z \leq 100$, umfasst.

Dieser Katalysator für die Hydrierung zeichnet sich dadurch aus, dass die Komponente Kupferoxid, die Komponente Aluminiumoxid und die Komponente Lanthanoxid, bevorzugt mit einer Sodalösung, simultan oder nacheinander gefällt werden, im Anschluss getrocknet, calciniert, verformt, z.B. tablettiert, und nochmals calciniert wird.

Kupferoxid bedeutet CuO, $Cu_2O$ oder ein Gemisch aus beiden Oxiden. Bei Mengenangaben wird Kupfer-(I)-oxid als Kupfer-(II)-oxid berechnet.

Aluminiumoxid bedeutet $Al_2O_3$ und Lanthanoxid bedeutet $La_2O_3$.

Insbesondere kommt folgende Fällungsmethode in Betracht:

A) Eine Kupfersalzlösung, eine Aluminiumsalzlösung und eine Lösung eines Salzes des Lanthans oder eine Lösung, enthaltend Kupfer-, Aluminium- und Lanthan-Salz, wird simultan bzw. werden nacheinander mit einer Sodalösung gefällt.
B) Fällung einer Kupfersalzlösung und, separat, einer Lösung eines Salzes des Lanthans oder einer Lösung, enthaltend Kupfersalz und ein Salz des Lanthans, auf einen vorgefertigten Aluminiumoxidträger. Dieser liegt in einer besonders bevorzugten Ausführungsform als Pulver in einer wässrigen Suspension vor. Das Trägermaterial kann aber z.B. auch als Kugeln, Stränge, Splitt oder Tabletten vorliegen.

In einer besonderen Ausführungsform zu B) (B1) wird eine Kupfersalzlösung und eine Lösung eines Salzes des Lanthans oder eine Lösung, enthaltend Kupfersalz und ein Salz des Lanthans, bevorzugt mit Sodalösung, gefällt. Als Vorlage wird eine wässrige Suspension des Trägermaterials Aluminiumoxid verwendet.

Ausgefällte Niederschläge, die aus A) oder B) resultieren, werden in üblicher Weise abgetrennt, z.B. filtriert, und vorzugsweise alkalifrei gewaschen, wie dies beispielsweise in der DE 198 09 418 A1 (BASF AG) beschrieben ist.

Nach der Fällung der Komponenten, besonders die Endprodukte aus A) oder aus B), werden diese bei erhöhter Temperatur, besonders bei Temperaturen von 50 bis 150 °C, vorzugsweise bei 110 bis 130 °C, getrocknet (z.B. über einen Zeitraum von 5 bis 30 Stunden, bevorzugt 10 bis 20 Stunden) und im Anschluss, vorzugsweise z.B. über einen Zeitraum von 0,5 bis 6 Stunden, besonders 1 bis 3 Stunden, bei im Allgemeinen 200 bis 700 °C, insbesondere bei 400 bis 650 °C, calciniert.

Als Ausgangssubstanzen für A) und/oder B) können prinzipiell alle in den bei der Fällung verwendeten Lösungsmitteln (bevorzugt ist Wasser) löslichen Cu(I)- und/oder Cu(II)-Salze, wie beispielsweise Nitrate, Carbonate, Acetate, Oxalate oder Ammonium-Komplexe, sowie analoge Aluminiumsalze und Salze des Lanthans verwendet werden. Besonders bevorzugt wird als Kupfersalz Kupfer-(II)-nitrat eingesetzt. Als Lanthansalz wird bevorzugt Lanthannitrat eingesetzt. Als Aluminiumsalz wird bevorzugt Aluminiumnitrat eingesetzt.

Die Zusammensetzung des oxidischen Materials ist bevorzugt so beschaffen, dass der Anteil an Kupferoxid im Bereich von 50 bis 80 Gew.-%, besonders 55 bis 75 Gew.-%, jeweils berechnet als CuO, der Anteil an Lanthanoxid im Bereich von 2 bis 20 Gew.-%, besonders 3 bis 15 Gew.-%, und der Anteil an Aluminiumoxid im Bereich 15 bis 35 Gew.-%, besonders 20 bis 30 Gew.-%, für alle Komponenten jeweils bezogen auf das Gesamtgewicht der Summe der oben genannten oxidischen Bestandteile, liegt, wobei diese drei Oxide zusammen mindestens 80 Gew.-%, besonders mindestens 95 Gew.-%, des oxidischen Materials nach Calcinierung darstellen, wobei gegebenfalls zugefügter Zement,

z.B. Tonerdezement, nicht dem oxidischen Material in obigem Sinne zugerechnet wird.

Eine bevorzugte Ausführungsform ist dadurch gekennzeichnet ist, dass das oxidische Material

(a) Kupferoxid mit einem Anteil im Bereich von $50 \leq x \leq 80$ Gew.-%, vorzugsweise $55 \leq x \leq 75$ Gew.-%, jeweils berechnet als CuO,
(b) Aluminiumoxid mit einem Anteil im Bereich von $15 \leq y \leq 35$ Gew.-%, vorzugsweise $20 \leq y \leq 30$ Gew.-%, und
(c) Lanthanoxid mit einem Anteil im Bereich von $2 \leq z \leq 20$ Gew.-%, bevorzugt $3 \leq z \leq 15$ Gew.-%, weiter bevorzugt $3{,}5 \leq z \leq 10$ Gew.-%,

jeweils bezogen auf das Gesamtgewicht des oxidischen Materials nach Calcinierung, wobei gilt: $80 \leq x + y + z \leq 100$, insbesondere $95 \leq x + y + z \leq 100$, umfasst.

Diese im erfindungsgemäßen Verfahren eingesetzten Katalysatoren zeichnen sich auch dadurch aus, dass die Zugabe des Lanthansalzes bei der Fällung zu einer hohen Stabilität des schließlich resultierenden Formkörpers, der als Katalysator eingesetzt wird, führt.

Dem oxidischen Material wird/werden anschließend (Schritt ii) pulverförmiges Kupfer und/oder Kupferblättchen und optional Graphit zugegeben. Bevorzugt wird pulverförmiges Kupfer und Graphit zugegeben. Die Zugabe von Graphit kann auch vor der Kupferzugabe erfolgen, wobei dann bevorzugt zunächst eine Vorkompaktierung durchgeführt wird. Z.B. wird Graphit in Mengen im Bereich von 0 bis 5 Gew.-%, bevorzugt im Bereich von 0,5 bis 4 Gew.-%, besonders bevorzugt im Bereich von 0,8 bis 2 Gew.-%, jeweils bezogen auf das Gesamtgewicht des oxidischen Materials nach Calicinierung, zugesetzt.

Als pulverförmiges Kupfer wird bevorzugt solches eingesetzt, welches einen Korndurchmesser im Bereich von 1 bis 700 µm, vorzugsweise im Bereich von 5 bis 500 µm, aufweist. Besonders bevorzugt wird ein pulverförmiges Kupfer eingesetzt, bei dem die Siebanalyse einen Anteil an Partikeln > 500 µm von ≤ 6 %, besonders einen Anteil an Partikeln > 350 µm von ≤ 5 %, ergibt. Die Korn-Morphologie ist bevorzugt kugelförmig.

Als Kupferblättchen werden bevorzugt solche eingesetzt, die einen D 50 - Wert im Bereich von 5 bis 40 µm, besonders im Bereich von 10 bis 35 µm, aufweisen (‚D 50 - Wert' bedeutet, dass 50 % der Partikel kleiner sind als der angegebene Wert). Bevorzugt ergibt die Siebanalyse einen Anteil an Partikeln > 45 µm von ≤ 6 %, besonders ≤ 2 %. Die Kupferblättchen weisen bevorzugt eine lamellare Blättchenstruktur auf.

Bevorzugt werden pulverförmiges Kupfer und/oder Kupferblättchen zusammengenommen in Mengen im Bereich von 0,5 bis 40 Gew.-%, bevorzugt im Bereich von 2 bis 20 Gew.-%, besonders bevorzugt im Bereich von 3 bis 10 Gew.-%, jeweils bezogen auf das Gesamtgewicht des oxidischen Materials nach Calicinierung, zugesetzt.

In besonderen Ausführungsformen kann das oxidische Material in einem Anteil von höchstens 10 Gew.-%, bevorzugt höchstens 5 Gew.-%, bezogen auf das Gesamtgewicht des oxidischen Materials nach Calcinierung, mindestens eine weitere Komponente aufweisen, die ausgewählt wird aus der Gruppe bestehend aus Oxiden der Elemente Re, Fe, Ru, Co, Rh, Ir, Ni, Pd und Pt. In Schritt iii wird das aus Schritt ii resultierende Gemisch zu einem Formkörper verformt und danach calciniert. Bevorzugt wird dem Gemisch vor dem Verformen zum Formkörper Graphit zugesetzt. Vorzugsweise wird so viel Graphit zugegeben, dass die Verformung zu einem Formkörper besser durchgeführt werden kann. In einer bevorzugten Ausführungsform werden 0,5 bis 5 Gew.-%, besonders 1 bis 3 Gew.-%, Graphit, bezogen auf das Gesamtgewicht des aus Schritt ii resultierende Gemisches, zugegeben.

Die Summe der Anteile aus oxidischem Material, metallischem Kupferpulver und/oder Kupferblättchen und ggf. Graphit ergibt bevorzugt mindestens 95 Gew.-%, besonders mindestens 98 Gew.-%, des Katalysator-Formkörpers.

Die Verformung in Schritt iii führt bevorzugt zu Tabletten, Ringen, Ringtabletten, Extrudaten, Wabenkörpern oder ähnlichen Formkörpern. Hierfür eignen sich sämtliche aus dem Stand der Technik bekannte Verfahren.

Im Anschluss an die Verformung erhaltene Formkörper werden nochmals, mindestens einmal, calciniert. Die Calcinierung erfolgt jeweils bevorzugt über eine Zeit von im allgemeinen 0,5 bis 10 Stunden (h), besonders 0,5 bis 2,5 Stunden. Die Temperatur bei diesem mindestens einen Calcinierungsschritt (und auch bei den optionalen Wiederholungs-Calcinierungsschritten) liegt im Allgemeinen im Bereich von 200 bis 600 °C, bevorzugt im Bereich von 250 bis 500 °C und besonders bevorzugt im Bereich von 270 bis 400 °C.

In einer weiteren Ausführung kann der erhaltene Formkörper auch noch mit siedendem Wasser und/oder Wasserdampf behandelt werden, bevor er für die Hydrierung eingesetzt wird.

Bei Einsatz als Katalysator in der oxidischen Form wird der Formkörper vor Beschickung mit den Edukten mit reduzierenden Gasen, beispielsweise Wasserstoff, vorzugsweise Wasserstoff/Inertgas-Gemischen, insbesondere Wasserstoff/Stickstoff-Gemischen, bei erhöhten Temperaturen, z.B. im Bereich von 100 bis 500 °C, bevorzugt im Bereich von 150 bis 350 °C und insbesondere im Bereich von 180 bis 200 °C, vorreduziert. Bevorzugt wird dabei ein Gasgemisch mit einem Wasserstoffanteil im Bereich von 1 bis 100 Vol.-%, besonders bevorzugt im Bereich von 1 bis 50 Vol.-%, verwendet.

In einer bevorzugten Ausführungsform wird der Formkörper vor dem Einsatz als Katalysator in an sich bekannter Weise durch Behandlung mit reduzierenden Medien aktiviert. Das Aktivieren erfolgt entweder vorab in einem Reduktionsofen oder nach dem Einbau im Reaktor. Ist der Reaktor vorab im Reduktionsofen aktiviert worden, wird er in den Reaktor eingebaut und direkt unter Wasserstoffdruck mit dem/den Edukt/en beschickt.

Nach einer katalytischen Herstellung von MEG wird zur Gewinnung des reinen Verfahrensprodukts MEG der Katalysator vom Rohprodukt abgetrennt. Viele Flüssigphasenhydrierungen von Oxalsäureestern setzen homogene Katalysatoren ein, die gegebenenfalls durch einen zusätzlichen Destillations-, Reaktions- oder Extraktionsschritt aus dem Rohprodukt entfernt werden müssen. Im vorliegenden Verfahren werden heterogene Katalysatoren verwendet, bei denen eine Abtrennung vom Rohprodukt leicht möglich ist. Eine Katalysatorabtrennung von der Reaktionslösung findet automatisch bei der bevorzugten Fahrweise im Festbettreaktor durch Rückhaltung des Katalysators im Reaktor statt. Nachfolgend ggf. auftretende Katalysatorpartikel durch Abrieb des Katalysators können zusätzlich durch ein Gitter, ein Netz, eine Fritte oder dem Fachmann bekannte Techniken entfernt werden.

Der vom Katalysator befreite Hydrieraustrag wird bevorzugt weiter aufgereinigt. Er enthält überwiegend das Hydrierprodukt Ethylenglykol und den/die Alkohole $R^1OH$, $R^2OH$, daneben, im Folgenden am Beispiel von DMO als Edukt I, in kleinen Mengen, jeweils bezogen auf das Produkt Ethylenglykol, molar weniger als 7 %, bevorzugt molar weniger als 5 %, 2-Methoxyethanol, molar weniger als 2 %, bevorzugt molar weniger als 1 %, Glykolsäuremethylester, molar weniger als 5 %, bevorzugt molar weniger als 2 %, Ethanol, sowie ggf. weitere Produkte, zumeist aber unter 1 % molar, bevorzugt unter 0,5 % molar, jeweils bezogen auf Ethylenglykol, wie 1,2-Propandiol, 1,2-Butanol, 2,3-Butanol, Acetaldehyd, 1,2-Dimethoxyethan, 1,1-Dimethoxyethan, 2,2-Dimethoxypropan, Dimethylether, Essigsäuremethylester, Ester der Ameisensäure, und ggf. andere, mengenmäßig unbedeutende Verbindungen.

Bevorzugte Ausführungsformen der Aufreinigung von MEG werden nachstehend am Beispiel von eingesetztem DMO näher erläutert.

Bevorzugt durch destillative Abtrennung von Leichter- und Höhersiedern vom MEG, wird das Rohhydrierprodukt von Methanol, Methylglykolat und sonstigen Verunreinigungen befreit. Bevorzugt wird die Aufreinigung von MEG in zwei, besonders mindestens drei Destillationskolonnen durchgeführt.

Der Hydrieraustrag wird in einer ersten Kolonne von Hochsiedern (Höhersieder als MEG) befreit, wobei über Kopf MeOH, Ethanol das ggf. noch Acetaldehyd, Dimethoxyethan und andere Leichtsieder enthalten kann, erhalten werden. Die Einspeisung des Stroms erfolgt im Verstärkungsteil der Kolonne, der Druck liegt bevorzugt zwischen 0,5 und 4 bar, bevorzugt zwischen 1 und 3 bar, besonders bevorzugt zwischen 1 und 2,5 bar. Die Temperatur beträgt bevorzugt 25-300 °C, bevorzugt 60-250 °C. Der Kopfstrom, hauptsächlich bestehend aus Methanol, kann anschließend separat aufgetrennt und das Methanol recycliert werden, wenn dies gewünscht ist, entweder durch Rückführung in den Prozess oder in einen mit im Verbund angeschlossenen Synthesereaktor zur Herstellung von Dimethyloxalat. Das Sumpfprodukt, welches hauptsächlich MEG und weitere Nebenprodukte wie z.B. Methylglykolat oder Ethanol enthält, wird in einer weiteren Kolonne, die bevorzugt bei niedrigerem Druck betrieben wird als die erste Kolonne, eingebracht. Hier werden Methylglykolat sowie Ethanol und darin enthaltene Verbindungen über Kopf abgetrennt und, bei Reaktor-Umlauf-Fahrweise, vorzugsweise mit dem Umlauf in den Reaktor zurückgeführt. Der Druck der zweiten Kolonne liegt zwischen 0,05 und 1 bar, bevorzugt zwischen 0,1 und 0,8 bar, besonders bevorzugt zwischen 0,1 und 0,5 bar. Die Temperatur beträgt 25-250 °C, bevorzugt 60-200 °C. Das Sumpfprodukt, hauptsächlich bestehend aus MEG und Hochsiedern (Höhersieder als MEG), wie z.B. 1,2-Butandiol, wird in der dritten Kolonne von hochsiedenden Verunreinigungen, die über den Sumpf ausgetragen werden, befreit. Der Druck der dritten Kolonne liegt zwischen 10 und 800 mbar, bevorzugt zwischen 25 und 500 mbar, besonders bevorzugt zwischen 35 und 350 mbar. Die Temperatur beträgt 25-250 °C, bevorzugt 80-200 °C. Nach der dritten Kolonne weist das MEG eine Reinheit von 99,9 Gew.-% auf. Falls dies nicht gewährleistet werden kann, können noch weitere Trennstufen, z.B. eine oder mehrere Destillationskolonnen, folgen.

Die Kolonnen können unterschiedliche Einbauten wie z. B. Füllkörper, Blechpackungen, Gewebepackungen oder Böden aufweisen.

Alternative Aufreinigungskonzepte beinhalten beispielsweise die Abreicherung der Verunreinigungen mittels Membranfiltration. Ebenfalls möglich ist die Entfernung von Wasserspuren mittels konzentrierter Natron- oder Kalilauge. Nach diesen Wasserabtrennmethoden wird das MEG vorzugsweise in zumindest einer Kolonne weiter aufgereinigt.

Das Katalysatorschüttvolumen ($Liter_{Kat.}$) wurde mittels eines graduierten 250 ml Messzylinders (nicht konisch, Innendurchmesser 37 mm) ermittelt.

Die Katalysatorschüttdichte wurde durch Einfüllen von 100 ml eines Katalysators in einen 250 ml Messzylinder (nicht konisch, Innendurchmesser 37 mm) ermittelt. Die Masse des eingefüllten Katalysators wurde durch Differenzmessung des Messzylinders mit und ohne Katalysator auf einer Waage mit einer Belastung bis zu 4000 g und Ablesegenauigkeit von 0,01 g bestimmt. Aus der Masse und dem Volumen konnte die Schüttdichte des Katalysators bestimmt werden (Schüttdichte [kg/l] = Gewicht [g] / Volumen [ml]).

Alle Druckabgaben beziehen sich auf den Absolutdruck.

Beispiele

Die Analytik aller Nebenkomponenten erfolgte über GC-Flächenprozent [Fl.-%]. Der Anteil an Methylglykolat

und DMO wurde zusätzlich in GC-Gewichtsprozent erfasst. Die gaschromatographische Trennung wurde an einer festen Phase (Stabil-WAX, 60 m, ∅ = 320 μm) mit Wasserstoff als Trägergas (Fluss: 1,1 ml/Min.) und einem FID-Detektor durchgeführt.

Die Selektivität der Katalysatoren unter den Reaktionsbedingungen wurde ermittelt nach der Formel

$$Selektivität\ [\%] = \frac{Anteil\ (\text{MEG})}{Anteil\ (\text{EtOH, 2-OMe-EtOH, 1,2-Propandiol, 1,2-Butandiol, MEG})} \bullet 100$$

wobei der Anteil in Flächenprozent der GC-Analytik ausgedrückt wird. Die Anteile der möglichen Produkte im Nenner wurden summiert. (EtOH = Ethanol; 2-OMe-EtOH = 2-Methoxy-ethanol).

Der Umsatz des DMO berechnet sich nach der Formel:

$$Umsatz\ DMO\ [\%] = \left(1 - \frac{Gew.\text{-\%}\ (\text{DMO})_{Austrag}}{Gew.\text{-\%}\ (\text{DMO})_{Zulauf}}\right) \bullet 100$$

Die Teilreduktion im Austrag wird definiert nach der Formel

$$Teilreduktion\ [\%] = \frac{Gew.\text{-\%}\ (\text{Me-Glykolat})}{M\ (\text{Me-Glykolat})} \bullet \frac{M\ (\text{DMO})}{Gew.\text{-\%}\ (\text{DMO})_{Zulauf}} \bullet 100$$

(M = molare Masse; Me-Glykolat = Methylglykolat = Glykolsäuremethylester = $HO\text{-}CH_2\text{-}CO(O)\text{-}OCH_3$).

Vergleichsbeispiel 1 bei niedrigem Druck

Die verwendete Apparatur bestand aus einem Zulaufteil mit Vorratsgefäß und Pumpe, einem 77 cm langen Rohrreaktor mit 1,4 cm Innendurchmesser und außenliegender Doppelmantelölbeheizung bzw. Kühlung welche in Rieselfahrweise betrieben wurde, einem auf 6 °C gekühlten Abscheider sowie Frischgas- und Abgas-Einrichtungen. Das Mol-Verhältnis von Frischwasserstoff zu DMO betrug 16 : 1, wobei das überschüssige Gas als Abgas ausgeschleust wurde. Der Reaktor wurde im geraden Durchgang betrieben.

Der Reaktor wurde mit 10 ml eines Barium-dotierten Kupfer-Chromits (3 mm Tabletten) gefüllt. Das Katalysatorschüttvolumen von 100 g Katalysator betrug 81 ml. Der Katalysator ist ein Verkaufsprodukt von BASF SE mit der Bezeichnung ‚Cu 1155 T' (≤ 69 Gew.-% Chrom(III)oxid, ≤ 21 Gew.-% Kupferoxid, ≤ 10 Gew.-% Bariumoxid). Oberhalb und unterhalb des Katalysators wurden Steatitkugeln als Inertschüttung gefüllt. Nach Inertisierung mit Stickstoff wurde der Katalysator mit einer Stickstoff/Wasserstoff-Mischung bei Normaldruck aktiviert. Nach Aktivierung des Katalysators wurde als Reaktorzulauf eine Lösung mit der Zusammensetzung 9 Gew.-% DMO, 45 Gew.-% Methanol (MeOH) und 46 Gew.-% MEG eingesetzt.

Bei einer Katalysatorbelastung von 0,23 kg DMO • $Liter_{Kat.}^{-1}$ • $h^{-1}$ wurde 78 % des DMO umgesetzt ($Liter_{Kat.}$ = Katalysatorschüttvolumen). Bei einer Selektivität von max. 10 % wurden neben MEG als Hauptprodukt als weitere Komponenten 2-Methoxyethanol, Dimethylether, Methylformiat und Methylglykolat gefunden. Eine Abgasmessung ergab, dass der Großteil des umgesetzten DMO (mind. 85 Gew.-% bezogen auf umgesetztes DMO) in Form von CO und $CO_2$ im Abgas zu finden war.

Vergleichsbeispiel 2a

Die verwendete Apparatur bestand aus einem Zulaufteil mit Vorratsgefäß und Pumpe, einem 1,80 m langen Rohrreaktor mit 3,4 cm Innendurchmesser und außenliegender Doppelmantelölbeheizung bzw. Kühlung welche in Rieselfahrweise betrieben wurde, einem wassergekühltem ersten Abscheider, einem zweiten auf 6 °C gekühlten Abscheider, einer Umlaufpumpe sowie Frischgas- und Abgas-Einrichtungen. Das Mol-Verhältnis von Frischwasserstoff zu DMO betrug 13 : 1, wobei das überschüssige Gas als Abgas ausgeschleust wurde. Das Gewichtsverhältnis von Umlauf zu Zulauf betrug ca. 11-22 zu 1.

Der Reaktor wurde mit 50 ml eines CuO (67 Gew.-%) / $La_2O_3$ (5 Gew.-%) / $Al_2O_3$ - Katalysators (3 mm Tabletten) gefüllt. Das Katalysatorschüttvolumen von 100 g Katalysator betrug 62 ml. Der Katalysator wurde hergestellt analog WO 2007/006719 A1 (BASF AG), Seiten 13-14, Beispiel 1. Am Reaktoreingang wurden 15 ml Glaskugeln oberhalb des Katalysators als Inertschüttung gefüllt. Nach Inertisierung mit Stickstoff wurde der Katalysator mit einer Stickstoff/Wasserstoff-Mischung bei Normaldruck aktiviert. Nach Aktivierung des Katalysators wurde der Umlauf mit einer Lösung von

Ethylenglykol (10 Gew.-%) in Methanol in Betrieb genommen und der Zieldruck im Reaktor sowie die Zieltemperatur eingestellt. Als Zulauf wurden 25 Gew.-% DMO in MeOH zugeführt.

Die in den Abscheidern anfallenden flüssigen Reaktionsausträge wurden gesammelt und vereinigt und analysiert.

| Eintrag | 1 | 2 | 3 | 4 | 5 |
|---|---|---|---|---|---|
| Druck [bar] | 170 | 170 | 170 | 170 | 170 |
| Temperatur [°C] | 170 | 190 | 210 | 210 | 230 |
| Belastung [$kg_{DMO}$ • $Liter_{Kat.}^{-1}$ • $h^{-1}$] | 0,20 | 0,20 | 0,20 | 0,10 | 0,10 |
| Umlauf/Zulauf [g/g] | 16 | 15 | 11 | 21 | 22 |
| Querschnittsbelastung [m/s] | 1,9 | 1,7 | 1,3 | 1,2 | 1,3 |
| $H_2$/DMO [mol/mol] | 13 | 13 | 13 | 26 | 26 |
| Umsatz DMO [molar] | 34 | 44 | 65 | 92 | 100 |
| Teilreduktion (Methylglykolat) [molar] | 19 | 13 | 7 | 6 | < 1 |
| Selektivität MEG [%] | 84 | 88 | 95 | 97 | 81 |
| Zersetzung DMO [%] | 9 | 16 | 29 | 32 | 17 |
| 1,2-BDO [Fl.-%] | < 0,001 | < 0,001 | < 0,001 | < 0,001 | 0,33 |
| 1,2-PDO [Fl.-%] | < 0,001 | < 0,001 | 0,003 | 0,01 | 0,8 |
| EtOH [Fl.-%] | < 0,001 | 0,05 | 0,06 | 0,12 | 1,62 |
| 2-OMe-EtOH [Fl.-%] | 0,1 | 0,2 | 0,6 | 0,9 | 0,9 |
| (BDO = Butandiol, PDO = Propandiol). | | | | | |

Vergleichsbeispiel 2b

Das Beispiel 2a wurde bei höherem Druck wiederholt, zusätzlich wurde mit höherem Umlauf gefahren.

| Eintrag | 1 | 2 | 3 | 4 | 5 |
|---|---|---|---|---|---|
| Druck [bar] | 200 | 200 | 200 | 200 | 200 |
| Temperatur [°C] | 200 | 215 | 230 | 215 | 230 |
| Belastung [$kg_{DMO}$ • $Liter_{Kat.}^{-1}$ • $h^{-1}$] | 0,20 | 0,20 | 0,20 | 0,15 | 0,15 |
| Umlauf/Zulauf [g/g] | 38 | 35 | 28 | 40 | 33 |
| Querschnittsbelastung [m/s] | 4,3 | 4,0 | 3,2 | 3,4 | 2,8 |
| $H_2$/DMO [mol/mol] | 13 | 13 | 13 | 17 | 17 |
| Umsatz DMO [molar] | 99 | 99 | 100 | 100 | 100 |
| Teilreduktion (Methylglykolat) [molar] | 4 | 3 | 1 | 2 | 1 |
| Selektivität MEG [%] | 96 | 91 | 80 | 87 | 77 |
| Zersetzung DMO [%] | 49 | 42 | 28 | 30 | 18 |
| 1,2-BDO [Fl.-%] | 0,002 | 0,006 | 0,03 | 0,01 | 0,04 |
| 1,2-PDO [Fl.-%] | 0,02 | 0,07 | 0,43 | 0,20 | 0,51 |
| EtOH [Fl.-%] | 0,11 | 0,27 | 0,83 | 0,58 | 1,12 |
| 2-OMe-EtOH [Fl.-%] | 0,14 | 0,30 | 0,51 | 0,36 | 0,55 |

Erfindungsgemäßes Beispiel 3

Die verwendete Apparatur bestand aus einem Zulaufteil mit Vorratsgefäß und Pumpe, einem 4 m langen gewundenen Rohrreaktor mit 0,4 cm Innendurchmesser, welcher isotherm in Rieselfahrweise betrieben wurde, einem Abscheider, einer Umlaufpumpe sowie Frischgas- und Abgas-Einrichtungen. Das Mol-Verhältnis von Frischwasserstoff zu DMO betrug 10 : 1, wobei das überschüssige Gas als Abgas ausgeschleust wurde. Das Massenverhältnis von Umlauf zu Zulauf betrug 10 : 1.

Der Reaktor wurde mit 75 g eines CuO (67 Gew.-%) / $La_2O_3$ (5 Gew.-%) / $Al_2O_3$-Katalysators (3 mm Tabletten), dem gleichen Katalysator wie in den Beispielen 2, und Inertmaterial (3 mm Glaskugeln), ebenfalls wie in den Beispielen 2, gefüllt. Nach Inertisierung mit Stickstoff wurde der Katalysator mit einer Stickstoff/Wasserstoff-Mischung bei Normaldruck aktiviert. Nach Aktivierung des Katalysators wurde der Umlauf mit einer Lösung von Ethylenglykol (10 Gew.-%) in Methanol in Betrieb genommen und der Zieldruck im Reaktor sowie die Zieltemperatur eingestellt. Als Zulauf wurden 15 Gew.-% DMO in MeOH zugeführt.

Die in den Abscheidern anfallenden flüssigen Reaktionsausträge wurden gesammelt und vereinigt und analysiert. Das Abgas wurde mittels Online-Analytik spektroskopisch auf seinen CO- und $CO_2$-Gehalt untersucht.

| Eintrag | 1 | 2 | 3 | 4 |
|---|---|---|---|---|
| Druck [bar] | 250 | 250 | 250 | 250 |
| Temperatur [°C] | 210 | 220 | 220 | 230 |
| Belastung [$kg_{DMO} \cdot Liter_{Kat.}^{-1} \cdot h^{-1}$] | 0,32 | 0,31 | 0,64 | 0,74 |
| Umlauf/Zulauf [g/g] | 10 | 10 | 10 | 10 |
| Querschnittsbelastung [m/s] | 109 | 109 | 219 | 255 |
| $H_2$/DMO [mol/mol] | 10 | 10 | 10 | 10 |
| Umsatz DMO [molar] | 92 | 99 | 98 | 97 |
| Teilreduktion (Methylglykolat) [molar] | 5 | 9 | 16 | 22 |
| Selektivität MEG [%] | 96 | 96 | 97 | 96 |
| Raum-Zeit-Ausbeute [$kg_{MEG} \cdot Liter_{Kat.}^{-1} \cdot h^{-1}$] | 0,13 | 0,15 | 0,31 | 0,31 |
| Zersetzung DMO [%] | 6 | 7 | 5 | 7 |
| 1,2-BDO [FI.-%] | 0,005 | < 0,001 | < 0,001 | < 0,001 |
| 1,2-PDO [FI.-%] | 0,012 | 0,015 | < 0,001 | < 0,001 |
| EtOH [FI.-%] | 0,12 | 0,19 | 0,12 | 0,09 |
| 2-OMe-EtOH [FI.-%] | 0,04 | 0,06 | 0,07 | 0,07 |

Der Rohaustrag wurde bei unvollständigem Umsatz anschließend noch über einen Nachreaktor gefahren, um zu einem vollständigen Umsatz zu gelangen. Die Selektivitäten dazu waren identisch zur Reaktion im Hauptreaktor.

## Patentansprüche

1. Verfahren zur Herstellung von Monoethylenglykol (MEG) durch metallkatalysierte Umsetzung eines Oxalsäuredialkylesters der Formel I

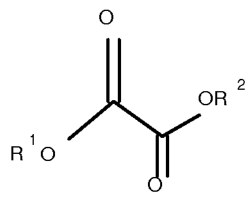

(I),

in der $R^1$ und $R^2$, jeweils unabhängig voneinander, Methyl, Ethyl, n-Propyl oder iso-Propyl bedeuten, mit Wasserstoff ($H_2$), **dadurch gekennzeichnet, dass** man den Oxalsäuredialkylester (I) als Schmelze oder gelöst in einem Lösungsmittel einsetzt, Oxalsäuredialkylester (I) und $H_2$ in einem molaren Verhältnis $H_2$ : Oxalsäuredialkylester (I) im Bereich von 4,0 bis 30 einsetzt, und die Umsetzung kontinuierlich in einem Reaktor bei einer Querschnittsbelastung von ≥ 10 m/s, einer Temperatur im Bereich von 150 bis 270 °C, einem Druck im Bereich von 150 bis 390 bar und in Gegenwart eines chromfreien Heterogenkatalysators, der Kupfer enthält, durchführt.

**2.** Verfahren nach Anspruch 1, **dadurch gekennzeichnet, dass** man die Umsetzung kontinuierlich bei einer Katalysatorbelastung im Bereich von 0,01 bis 5,0 kg Oxalsäuredialkylester (I) • $Liter_{Kat.}^{-1}$ • $h^{-1}$ durchführt.

**3.** Verfahren nach dem vorhergehenden Anspruch, **dadurch gekennzeichnet, dass** man die Katalysatorbelastung derart einstellt, dass der Umsatz an Oxalsäuredialkylester (I) ≥ 90 % beträgt.

**4.** Verfahren nach einem der vorhergehenden Ansprüche, **dadurch gekennzeichnet, dass** man die Umsetzung kontinuierlich in einem Reaktor bei einer Querschnittsbelastung im Bereich von ≥ 10 bis 1000 m/s durchführt.

**5.** Verfahren nach einem der vorhergehenden Ansprüche 1 bis 3, **dadurch gekennzeichnet, dass** man die Umsetzung kontinuierlich in einem Reaktor bei einer Querschnittsbelastung im Bereich von > 10 bis 500 m/s durchführt.

**6.** Verfahren nach einem der vorhergehenden Ansprüche, **dadurch gekennzeichnet, dass** man Oxalsäuredialkylester (I) und $H_2$ in einem molaren Verhältnis $H_2$ : Oxalsäuredialkylester (I) im Bereich von 4,2 bis 20 einsetzt.

**7.** Verfahren nach einem der vorhergehenden Ansprüche 1 bis 5, **dadurch gekennzeichnet, dass** man Oxalsäuredialkylester (I) und $H_2$ in einem molaren Verhältnis $H_2$ : Oxalsäuredialkylester (I) im Bereich von 4,3 bis 15 einsetzt.

**8.** Verfahren nach einem der vorhergehenden Ansprüche, **dadurch gekennzeichnet, dass** man die Umsetzung bei einer Temperatur im Bereich von 170 bis 260 °C durchführt.

**9.** Verfahren nach einem der vorhergehenden Ansprüche, **dadurch gekennzeichnet, dass** man die Umsetzung bei einem Druck im Bereich von 160 bis 290 bar durchführt.

**10.** Verfahren nach einem der vorhergehenden Ansprüche, **dadurch gekennzeichnet, dass** man die Umsetzung in einem Reaktor kontinuierlich durchführt und der Reaktor mit einem Umlauf betrieben wird, indem ein Teil des Reaktoraustrags vom Reaktorausgang zum Reaktoreingang zurückgeführt wird.

**11.** Verfahren nach einem der vorhergehenden Ansprüche, **dadurch gekennzeichnet, dass** es sich bei dem Oxalsäuredialkylester (I) um Oxalsäuredimethylester handelt.

**12.** Verfahren nach einem der vorhergehenden Ansprüche, **dadurch gekennzeichnet, dass** der kupferhaltige Heterogenkatalysator Aluminiumoxid enthält.

**13.** Verfahren nach einem der vorhergehenden Ansprüche, **dadurch gekennzeichnet, dass** der Heterogenkatalysator Lanthanoxid enthält.

**14.** Verfahren nach einem der vorhergehenden Ansprüche, **dadurch gekennzeichnet, dass** der Heterogenkatalysator 10 bis 80 Gew.-% Kupfer, 0,5 bis 20 Gew.-% Lanthanoxid und 19,5 bis 89,5 Gew.-% Aluminiumoxid enthält.

**15.** Verfahren nach einem der vorhergehenden Ansprüche, **dadurch gekennzeichnet, dass** es sich bei dem Lösungsmittel um Methanol, Ethanol, n-Propanol, iso-Propanol und/oder Ethylenglykol handelt.

**16.** Verfahren nach einem der vorhergehenden Ansprüche, **dadurch gekennzeichnet, dass** es sich bei dem Reaktor um einen Rohrbündelreaktor oder Schachtreaktor handelt.

**17.** Verfahren nach einem der vorhergehenden Ansprüche, **dadurch gekennzeichnet, dass** der Katalysator im Reaktor als Festbett angeordnet ist.

**Claims**

1. A process for preparing monoethylene glycol (MEG) by metal-catalyzed reaction of a dialkyl oxalate of the formula I

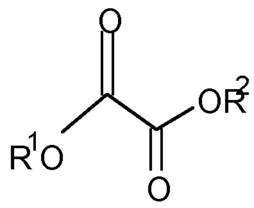

(I),

where $R^1$ and $R^2$ are each, independently of one another, methyl, ethyl, n-propyl or isopropyl, with hydrogen ($H_2$), wherein the dialkyl oxalate (I) is used as melt or as a solution in a solvent, dialkyl oxalate (I) and $H_2$ are used in a molar ratio of $H_2$ : dialkyl oxalate (I) in the range from 4.0 to 30 and the reaction is carried out continuously in a reactor at a cross-sectional loading of ≥ 10 m/s, a temperature in the range from 150 to 270°C, a pressure in the range from 150 to 390 bar and in the presence of a chromium-free heterogeneous catalyst comprising copper.

2. The process according to claim 1, wherein the reaction is carried out continuously at a space velocity over the catalyst in the range from 0.01 to 5.0 kg of dialkyl oxalate (I) • $liter_{cat.}^{-1}$ • $h^{-1}$.

3. The process according to the preceding claim, wherein the space velocity over the catalyst is set so that the conversion of dialkyl oxalate (I) is ≥ 90%.

4. The process according to any of the preceding claims, wherein the reaction is carried out continuously in a reactor at a cross-sectional loading in the range from ≥ 10 to 1000 m/s.

5. The process according to any of the preceding claims 1 to 3, wherein the reaction is carried out continuously in a reactor at a cross-sectional loading in the range from > 10 to 500 m/s.

6. The process according to any of the preceding claims, wherein dialkyl oxalate (I) and $H_2$ are used in a molar ratio of $H_2$ : dialkyl oxalate (I) in the range from 4.2 to 20.

7. The process according to any of claims 1 to 5, wherein dialkyl oxalate (I) and $H_2$ are used in a molar ratio of $H_2$ : dialkyl oxalate (I) in the range from 4.3 to 15.

8. The process according to any of the preceding claims, wherein the reaction is carried out at a temperature in the range from 170 to 260°C.

9. The process according to any of the preceding claims, wherein the reaction is carried out at a pressure in the range from 160 to 290 bar.

10. The process according to any of the preceding claims, wherein the reaction is carried out continuously in a reactor and the reactor is operated with recycle by part of the reactor output being recirculated from the reactor outlet to the reactor inlet.

11. The process according to any of the preceding claims, wherein the dialkyl oxalate (I) is dimethyl oxalate.

12. The process according to any of the preceding claims, wherein the copper-comprising heterogeneous catalyst comprises aluminum oxide.

13. The process according to any of the preceding claims, wherein the heterogeneous catalyst comprises lanthanum oxide.

14. The process according to any of the preceding claims, wherein the heterogeneous catalyst comprises from 10 to 80% by weight of copper, from 0.5 to 20% by weight of lanthanum oxide and from 19.5 to 89.5% by weight of

aluminum oxide.

**15.** The process according to any of the preceding claims, wherein the solvent is methanol, ethanol, n-propanol, isopropanol and/or ethylene glycol.

**16.** The process according to any of the preceding claims, wherein the reactor is a shell-and-tube reactor or shaft reactor.

**17.** The process according to any of the preceding claims, wherein the catalyst is arranged as a fixed bed in the reactor.

**Revendications**

**1.** Procédé pour la préparation de monoéthylèneglycol (MEG) par transformation catalysée par un métal d'un ester de dialkyle d'acide oxalique de formule

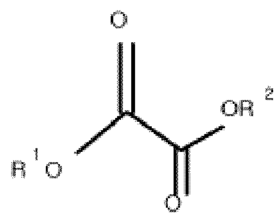

(I),

dans laquelle $R^1$ et $R^2$, à chaque fois indépendamment l'un de l'autre, signifient méthyle, éthyle, n-propyle ou isopropyle,
avec de l'hydrogène ($H_2$), **caractérisé en ce qu'**on utilise l'ester de dialkyle d'acide oxalique (I) en tant que masse fondue ou dissous dans un solvant, on utilise l'ester de dialkyle d'acide oxalique (I) et $H_2$ en un rapport molaire $H_2$ : ester de dialkyle d'acide oxalique (I) dans la plage de 4,0 à 30, et on met en œuvre la transformation en continu dans un réacteur à une charge sur la section transversale de ≥ 10 m/s, à une température dans la plage de 150 à 270 °C, à une pression dans la plage de 150 à 390 bars et en présence d'un catalyseur hétérogène exempt de chrome, qui contient du cuivre.

**2.** Procédé selon la revendication 1, **caractérisé en ce qu'**on met en œuvre la transformation en continu à une charge de catalyseur dans la plage de 0,01 à 5,0 kg d'ester de dialkyle d'acide oxalique (I) •$litre_{cat.}^{-1}$•$h^{-1}$.

**3.** Procédé selon la revendication précédente, **caractérisé en ce qu'**on ajuste la charge de catalyseur de telle sorte que la transformation de l'ester de dialkyle d'acide oxalique (I) est ≥ 90 %.

**4.** Procédé selon l'une quelconque des revendications précédentes, **caractérisé en ce qu'**on met en œuvre la transformation en continu dans un réacteur à une charge sur la section transversale dans la plage de ≥ 10 à 1 000 m/s.

**5.** Procédé selon l'une quelconque des revendications 1 à 3, **caractérisé en ce qu'**on met en œuvre la transformation en continu dans un réacteur à une charge sur la section transversale dans la plage de > 10 à 500 m/s.

**6.** Procédé selon l'une quelconque des revendications précédentes, **caractérisé en ce qu'**on utilise l'ester de dialkyle d'acide oxalique (I) et $H_2$ en un rapport molaire $H_2$ : ester de dialkyle d'acide oxalique (I) dans la plage de 4,2 à 20.

**7.** Procédé selon l'une quelconque des revendications 1 à 5, **caractérisé en ce qu'**on utilise l'ester de dialkyle d'acide oxalique (I) et $H_2$ en un rapport molaire $H_2$ : ester de dialkyle d'acide oxalique (I) dans la plage de 4,3 à 15.

**8.** Procédé selon l'une quelconque des revendications précédentes, **caractérisé en ce qu'**on met en œuvre la transformation à une température dans la plage de 170 à 260 °C.

**9.** Procédé selon l'une quelconque des revendications précédentes, **caractérisé en ce qu'**on met en œuvre la transformation à une pression dans la plage de 160 à 290 bars.

10. Procédé selon l'une quelconque des revendications précédentes, **caractérisé en ce qu'**on met en œuvre la transformation dans un réacteur en continu et le réacteur fonctionne en circulation par le fait qu'une partie du produit d'évacuation du réacteur est reconduite de la sortie du réacteur à l'entrée du réacteur.

11. Procédé selon l'une quelconque des revendications précédentes, **caractérisé en ce que** l'ester de dialkyle d'acide oxalique (I) est l'ester de diméthyle d'acide oxalique.

12. Procédé selon l'une quelconque des revendications précédentes, **caractérisé en ce que** le catalyseur hétérogène contenant du cuivre contient de l'oxyde d'aluminium.

13. Procédé selon l'une quelconque des revendications précédentes, **caractérisé en ce que** le catalyseur hétérogène contient de l'oxyde de lanthane.

14. Procédé selon l'une quelconque des revendications précédentes, **caractérisé en ce que** le catalyseur hétérogène contient 10 à 80 % en poids de cuivre, 0,5 à 20 % en poids d'oxyde de lanthane et 19,5 à 89,5 % en poids d'oxyde d'aluminium.

15. Procédé selon l'une quelconque des revendications précédentes, **caractérisé en ce que** le solvant est le méthanol, l'éthanol, le n-propanol, l'isopropanol et/ou l'éthylène glycol.

16. Procédé selon l'une quelconque des revendications précédentes, **caractérisé en ce que** le réacteur est un réacteur à faisceau tubulaire ou un réacteur en forme de cage.

17. Procédé selon l'une quelconque des revendications précédentes, **caractérisé en ce que** le catalyseur est disposé en tant que lit fixe dans le réacteur.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4453026 A
- US 2060880 A
- DE 3843956 A1
- CN 101475443 A
- US 20100179356 A1, J. Liu
- DE 19809418 A1
- WO 2007006719 A1


### In der Beschreibung aufgeführte Nicht-Patentliteratur


- **L. ZHUXIA et al.** *Huaxue fanying gongcheng yu gongyi (Chemical Reaction Engineering and Technology),* 2004, vol. 20, 121-128
- **Y. WANG et al.** *Catal. Sci. Technol.,* 2012, vol. 2, 1637-1639
- **H. YUE et al.** *Chem. Soc. Rev.,* 2012, vol. 41, 4218-4244
- **H.T. TEUNISSEN et al.** *Chem. Commun.,* 1997, 667-668